# FASCICULE DE BREVET EUROPEEN

(11) **EP 0 646 033 B1**
(45) Date de publication et mention de la délivrance du brevet: **07.01.1998**
(21) Numéro de dépôt: 93913102.5
(22) Date de dépôt: 09.06.1993
(51) Int. Cl.: A61N 1/16

(54) **DISPOSITIF DE PROTECTION CONTRE LES EFFETS BIOLOGIQUES DES RAYONNEMENTS ELECTROMAGNETIQUES NON IONISANTS, NOTAMMENT CEUX EMIS PAR LES APPAREILS A ECRAN DE VISUALISATION**
EINRICHTUNG ZUM SCHUTZ GEGEN DIE BIOLOGISCHEN EINWIRKUNGEN VON NICHTIONISIERENDE ELEKTROMAGNETISCHE STRAHLUNGEN, INSBESONDERE DIE DIE VON ANZEIGEGERÄTEN AUSGESTRAHLT WERDEN
DEVICE FOR PROTECTION AGAINST THE BIOLOGICAL EFFECTS OF ELECTROMAGNETIC NON-IONISING RADIATIONS, PARTICULARLY THOSE EMITTED BY VDU EQUIPMENT

(30) Priorité: 10.06.1992 FR 9207171
(43) Date de publication de la demande: 05.04.1995
(73) Titulaire: FILLION-ROBIN, Maurice Serge Guy, F-71390 Cersot (FR)
(72) Inventeur: FILLION-ROBIN, Maurice Serge Guy, F-71390 Cersot (FR)
(74) Mandataire: Dupuis-Latour, Dominique
(86) Numéro de dépôt international: FR9300546
(87) Numéro de publication internationale: WO9325270

(56) Documents cités:
- EP-A- 0 099 872
- FR-A- 941 382
- FR-A- 965 820
- FR-A- 2 580 180

## Description

L'invention concerne un dispositif de protection contre les effets biologiques des rayonnements électromagnétiques non ionisants, en particulier ceux émis par les appareils électriques ou électroniques domestiques, bureautiques ou industriels.

En particulier, les appareils à écran de visualisation (qu'il s'agisse notamment de moniteurs informatiques, de téléviseurs ou de terminaux vidéotex), sont réputés produire, du fait des rayonnements électromagnétiques qu'ils émettent, un certain nombre de nuisances biologiques révélées par divers troubles organiques tels que fatigue oculaire, maux de tête, états de stress, etc.

On a pu en effet montrer que les radiations lumineuses issues de l'écran (gêne due à l'intensité lumineuse, au contraste, à la concentration sur une image fixe ou encore à la fréquence de balayage) ne sont pas les seules causes de ces troubles organiques, mais que les rayonnements électromagnétiques (non ionisants) émis par ces appareils ont également des effets biologiques avérés.

L'invention a pour objet un dispositif permettant de se protéger à l'encontre de ces derniers effets biologiques, propres à l'existence d'un champ de rayonnement relativement intense régnant dans l'environnement d'utilisation de ces écrans de visualisation ― ou autres éléments semblables générateurs de champs électromagnétiques. En particulier, l'invention n'est pas limitée au cas particulier des appareils à écran de visualisation, mais est également destinée à être utilisée à l'encontre des champs électromagnétiques de toutes natures, créés par exemple par les fours à micro-ondes, les lignes à haute tension, les téléphones sans fil et téléphones cellulaires, les jeux vidéo, etc.

Divers dispositifs ont été proposés pour pallier les effets de ces radiations, mais ils se sont avérés en pratique peu efficaces, et parfois même dangereux car certains proposent d'utiliser des éléments qui produisent eux-mêmes des rayonnements non ionisants, par exemple à l'aide de barrettes ferreuses formant émetteur bipolaire, technique dont l'innocuité est loin d'être certaine.

Par ailleurs, la présomption d'efficacité sur l'être humain ou l'animal de la plupart des dispositifs proposés jusqu'ici n'a pas été vérifiée par des expérimentations biologiques ou biophysiques.

Le dispositif de protection de l'invention, au contraire, n'émet pas de tels rayonnements non ionisants et il a fait l'objet de tests biologique et biophysiques d'efficacité et d'innocuité.

Il est caractérisé en ce qu'il comprend un récipient, destiné à être disposé à proximité de la source desdits rayonnements, contenant un milieu ayant subi une irradiation préalable et un sel métallique ou une combinaison de sels métalliques portés par ce milieu à une concentration maximale de 20 mg de sel(s) pour 100 l de milieu, ledit récipient étant en un matériau non ferromagnétique et chimiquement neutre par rapport audit milieu et audit sel.

Avantageusement, ledit sel est un sel d'un métal alcalin ou alcatino-terreux et ledit milieu est un milieu liquide, notamment un milieu aqueux.

Ladite irradiation préalable du milieu peut être avantageusement réalisée par utilisation d'une antenne de Lecher placée au-dessus d'un volume dudit milieu et tenue à la main par l'une de ses branches, ou encore par utilisation d'une source de rayonnement cohérent associée à des moyens pour coopérer avec la main d'un opérateur.

Ledit récipient peut notamment avoir la forme d'au moins un tube allongé fermé à ses deux extrémités, par exemple de deux tubes parallèles disposés côte à côte, et de longueur comprise entre 10 et 25 cm.

On va maintenant décrire un exemple de réalisation du dispositif de l'invention, en référence aux dessins annexés.

La figure 1 est une vue perspective montrant la configuration générale du dispositif de l'invention.

La figure 2 est une coupe en élévation de ce même dispositif, qui en montre la structure interne.

La figure 3 montre un exemple de placement de ce dispositif sur un appareil émetteur de rayonnements vis-à-vis desquels on souhaite se protéger.

La figure 4 illustre une première forme de mise en oeuvre du procédé de préparation du liquide contenu dans le dispositif de l'invention.

La figure 5 illustre une autre forme de mise en oeuvre de ce même procédé.

Les figures 6 et 7 sont des diagrammes montrant les résultats d'études cliniques effectuées pour montrer l'efficacité du dispositif de l'invention.

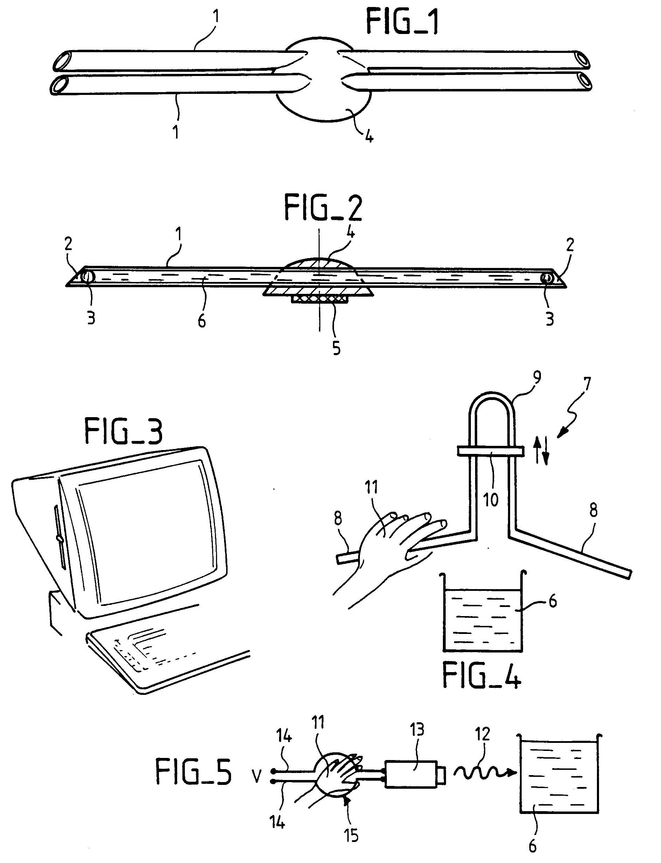

Sur les figures 1 et 2, on a représenté un dispositif de protection selon l'invention, qui est réalisé sous forme de deux tubes parallèles creux semblables 1, obturés à chacune de leurs extrémités 2 par un moyen approprié (par exemple une bille d'acier 3 enfoncée à force comme illustré sur la figure, ou encore par bouchage au moyen d'un élastomère réticulable, ou encore par sertissage mécanique, etc.). Ces tubes sont maintenus, par exemple dans une région centrale, par un support commun 4 permettant en outre la fixation du dispositif sur un endroit approprié de l'appareil dont on veut se protéger, par exemple au moyen d'une pastille d'adhésif double face 5. La figure 3 illustre un exemple de dispositif selon l'invention placé contre une paroi latérale d'un moniteur informatique, donc à proximité des sources de rayonnement de l'écran de visualisation de celui-ci.

Les tubes 1 sont remplis d'un liquide approprié 6 dont on décrira plus loin la composition. Les tubes 1 doivent être en un matériau non ferromagnétique et chimiquement neutre (non réactif) par rapport au liquide 6 ; il peuvent être en métal, par exemple en aluminium ou en un alliage d'aluminium, ou encore en une matière synthétique telle qu'un polyamide ou un polypropylène. Dans l'exemple illustré, chacun des tubes a un diamètre intérieur de 4 mm et un diamètre extérieur de 6 mm, avec une longueur hors-tout de 17 cm pour une longueur utile intérieure de 15 cm environ, ce qui donne à chaque tube une contenance de l'ordre de 1,5 à 2 cm³, valeur (non limitative) correspondant à la quantité de liquide nécessaire pour assurer une protection à l'encontre des rayonnements typiquement émis par un appareil de type moniteur d'ordinateur ou téléviseur.

On notera que la forme et les dimensions illustrées sur les figures 1 et 2, si elles sont avantageuses, ne sont pas essentielles pour la mise en oeuvre de l'invention, dont le principe essentiel réside dans la nature et le traitement du liquide 6. Ainsi, d'autres formes peuvent être envisagées, par exemple spirale, anneau, sphère creux définissant un volume intérieur ayant une capacité du même ordre de grandeur. De plus, si l'on prévoit deux tubes (ou deux anneaux, etc.), les matériaux des deux récipients peuvent être différents, dès lors qu'ils sont non ferromagnétiques et neutres à l'égard du liquide qu'ils contiennent.

On va maintenant décrire la manière dont est préparé le milieu liquide actif 6. On notera à cet égard que ce milieu actif n'est pas nécessairement présent sous forme liquide, mais peut l'être sous forme solide, gazeuse, gélifiée, etc.

On prend un milieu aqueux, par exemple de l'eau purifiée, et on le soumet à un traitement préalable d'irradiation non ionisante, par exemple, comme illustré figure 4, au moyen d'une antenne de Lecher : cette antenne 7 est placée au-dessus d'un récipient 6 contenant le milieu aqueux, et elle est tenue à la main par l'une de ses deux branches (tiges aimantées 8), tandis que la dimension de la bouche résonnante 9 est ajustée par la barrette mobile 10 à une fréquence de résonance comprise entre 0 et 10000 MHz ; l'antenne est maintenue par la main 11 de l'opérateur au-dessus du récipient 6 afin de réaliser, de manière en elle-même connue, un couplage résonnant entre l'opérateur et le milieu aqueux 6 et opérer ainsi une irradiation non ionisante de ce dernier.

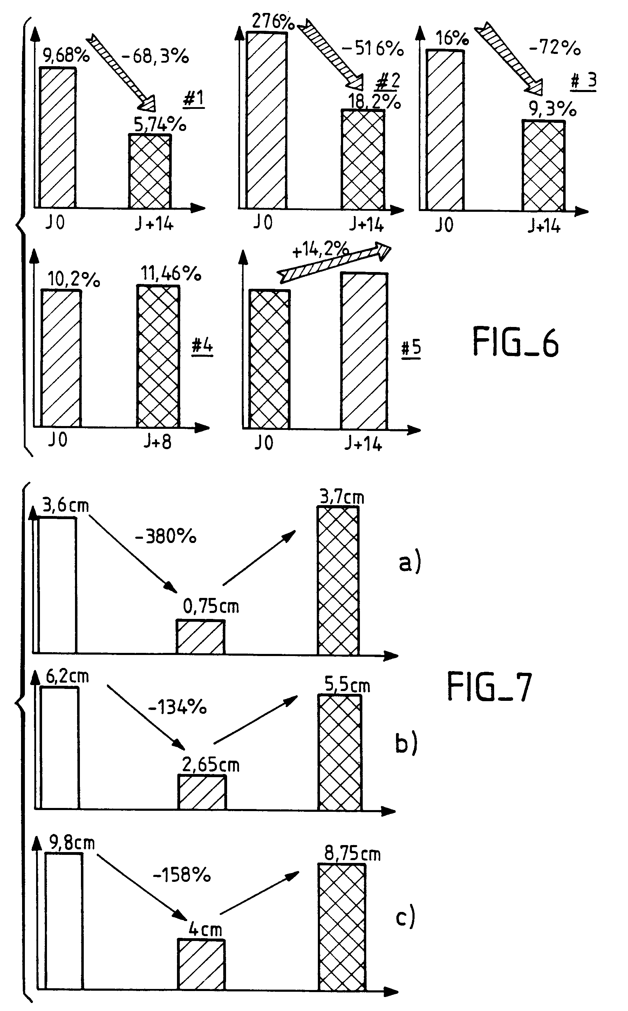

Une variante de mise en oeuvre de l'irradiation peut être réalisée, comme illustré figure 5, par application d'un rayonnement cohérent 12 émis par un laser 13 associé à des moyens pour coopérer avec la main 11 de l'opérateur, par exemple une bouche 15 formée dans la ligne d'alimentation 14 du laser 13 et dans laquelle l'opérateur passera sa main, de manière à assurer ici encore un couplage entre la main de l'opérateur et le milieu aqueux 6 contenu dans le récipient illuminé par le laser.

Le milieu aqueux ainsi préparé est additionné d'un sel métallique ou d'une combinaison de sels métalliques, à une concentration maximale de 20 mg de sel(s) pour 100 litres de milieu aqueux.

On notera que l'addition du sel peut indifféremment être réalisée avant ou après l'irradiation préalable non ionisante que l'on vient de décrire en référence aux figures 4 et 5.

Le sel métallique choisi est de préférence un sel d'un métal appartenant au groupe formé par Li, Na, Rb, K, Cs, Mg, Ca, Sr, Ba, Fe⁺⁺⁺, Co, Ni, Mn, Zn, Cd, Pb, Cu, Fe⁺⁺, Ag, Hg, en excluant de préférence les sels de terres rares et en choisissant avantageusement un sel d'un métal alcalin ou alcalino-terreux.

Par ailleurs, on notera que la valeur maximale de concentration du sel (ou des sels) indiquée plus haut est sensiblement indépendante de la nature du sel choisi.

On va maintenant donner un certain nombre de résultats d'essais cliniques montrant les résultats procurés par le dispositif de l'invention. Préalablement, on notera que, avec le dispositif décrit plus haut, l'effet de protection est obtenu jusqu'à plusieurs dizaines de centimètres, typiquement jusqu'à 3 à 4 mètres de rayon, permettant ainsi de protéger complètement l'utilisateur quelle que soit sa position par rapport à la source de rayonnement électromagnétique et quel que soit l'endroit où est placé le dispositif de l'invention à proximité de cette même source (ce dernier positionnement n'étant pas un paramètre critique pour l'obtention d'une protection efficace).

### 1°) Étude posturologique

On a étudié la position et l'évolution au sol de la projection du centre de gravité d'un individu placé sur une plate-forme de stabilométrie clinique normalisée (par exemple de type *Dynatronic DYN 50*) donnant en particulier une indication de la stabilité de l'individu et de sa latéralité par analyse de la surface (stabilité) et de la décentration (latéralité) de la trace de la projection du centre de gravité sur le statokinésigramme obtenu.

Un excès de surface et/ou une latéralité excessive sont habituellement corrélés à des pathologies telles que strabisme, troubles de la convergence oculaire, lombalgies, cervicalgies, sensations vertigineuses, etc.

Sur un échantillon de 35 personnes, la posture de chaque individu a été analysée pendant une durée de 50 secondes : d'abord sans ordinateur à proximité, ensuite avec un ordinateur placé à 70 cm mais sans dispositif selon l'invention (situation que l'on appellera par la suite "écran non protégé"), et enfin avec le même ordinateur muni d'un dispositif selon l'invention ("écran protégé").

On observe que dans 73,3 % des cas (dont 63,5 % de façon statistiquement significative, c'est-à-dire présentant un écart de latéralité d'au moins ± 7,1 mm), les individus devant l'écran protégé améliorent leur équilibre postural et éliminent ainsi un facteur de fatigue et de troubles organiques ou psychologiques pouvant être liés au travail sur écran.

Cette étude montre en outre que :
― un tiers des personnes ayant présenté l'amélioration précitée changent de latéralité gauche/droite,
― 46,5 % des personnes améliorent leur équilibre devant l'écran protégé par rapport à la mesure initiale effectuée sans écran,
― 40 % des individus aggravent leur déséquilibre en latéralité lorsqu'ils passent de la situation initiale sans écran à la situation avec écran non protégé, et
― il n'a jamais été constaté d'aggravation statistiquement significative de la latéralité du fait de l'adjonction du dispositif selon l'invention.

### 2°) Étude immunologique

Cette étude, effectuée sur cinq sujets, prend en compte le rapport lymphocytes Tₕₑₗₚₑᵣ/lymphocytes T_{inducteur}, notamment le taux CD₅/CD₂₀ qui, lorsqu'il s'accroît, est un indicateur de la tendance à développer des maladies auto-immunes et, lorsqu'il décroît, indique une amélioration du potentiel immunitaire. Un taux normal se situe autour de 10 %, un écart entre deux analyses étant considéré comme significatif pour une variation d'au moins ± 15 % par rapport au chiffre initial.

Les personnes testées étaient toutes utilisatrices d'écrans informatiques pendant plusieurs heures par jour.

Les analyses sanguines ont été réalisées comme suit :
- Sujets 1, 2, 3 :: première analyse avec écran non protégé, deuxième analyse avec écran protégé pendant 14 jours,
- Sujet 4 :: première analyse avec écran non protégé, deuxième analyse avec écran protégé pendant 7 jours,
- Sujet 5 :: première analyse avec écran protégé depuis plus de 3 mois, deuxième analyse après 14 jours de suppression de la protection.

Les résultats sont donnés sur les graphiques référencés #1 à #5 de la figure 6, respectivement pour les cinq sujets considérés ; les hachures simples correspondent à une situation avec écran non protégé et les hachures croisées, à une situation avec écran protégé par le dispositif de l'invention. On peut voir que, à l'exception du sujet n° 4 pour lequel la variation du taux CD₅/CD₂₀ n'est pas suffisamment significative, pour les autres sujets le passage d'une situation d'écran non protégé à une situation d'écran protégé montre une amélioration significative du potentiel immunitaire de l'individu. Inversement, dans le cas du sujet n° 5 la suppression de la protection a montré une dégradation du potentiel immunitaire.

### 3°) Étude de médecine énergétique

Dans cette étude, on a mesuré, par le réflexe auriculo-cardiaque selon la méthode Nogier, les variations quantitatives des champs énergétiques, exprimés en profondeur de pénétration, correspondants à un tissu superficiel, un tissu moyen et un tissu profond ; les valeurs respectives considérées comme normales sont typiquement de 6 cm, 8 cm et 10 cm, une valeur plus faible correspondant à une dégradation du potentiel de l'individu.

Des mesures ont été effectuées sur dix patients, successivement dans une situation sans écran, avec écran non protégé et avec écran protégé.

Les résultats sont illustrés sur les figures 7(a) à (c), respectivement pour un tissu superficiel, un tissu moyen et un tissu profond. L'absence de hachures correspond à la situation sans écran, des hachures simples correspondent à un écran non protégé et des hachures croisées à un écran protégé. Les valeurs portées sur les graphiques sont les valeurs moyennes des données recueillies pour l'ensemble de l'échantillon.

Comme on peut le voir très nettement à l'examen des graphiques de ces figures, on constate que les capacités potentielles de l'individu, fortement dégradées par une situation de travail sur écran non protégé, sont, selon le cas, intégralement ou presque totalement restaurées par l'utilisation du dispositif de l'invention.

### 4°) Étude neurophysiologique

On a étudié les paramètres neurophysiologiques d'un échantillon de patients travaillant quotidiennement sur terminal informatique. Ces patients travaillent tout d'abord sur des moniteurs non protégés ; au 4^{e} jour, on effectue une première étude neurophysiologique, au 7^{e} jour on place le dispositif de protection de l'invention et au 18^{e} jour on effectue une seconde étude neurophysiologique.

On constate entre les deux observations une nette amélioration de la réponse aux stimuli lumineux pour les séquences yeux fermés/yeux ouverts et, surtout, aux stimulations lumineuses à 20 Hz, d'une part pour la totalité (0,5-31,5 Hz) de la bande enregistrée par l'électroencéphalographe, d'autre part pour les bandes alpha (7,5 à 13,5 Hz) et bêta (13,5 à 31,5 Hz). Dans l'ensemble, cette augmentation va du simple au double.

## Revendications

1. Un dispositif de protection contre les effets biologiques des rayonnements électromagnétiques non ionisants, notamment ceux émis par les appareils à écran de visualisation, caractérisé en ce qu'il comprend un récipient (1), destiné à être disposé à proximité de la source desdits rayonnements, contenant un milieu (6) ayant subi une irradiation non ionisante préalable et un sel métallique ou une combinaison de sels métalliques portés par ce milieu à une concentration maximale de 20 mg de sel(s) pour 100 l de milieu, ledit récipient étant en un matériau non ferromagnétique et chimiquement neutre par rapport audit milieu et audit sel.

2. Le dispositif de la revendication 1, dans lequel ledit sel est un sel d'un métal alcalin ou alcalino-terreux.

3. Le dispositif de la revendication 1, dans lequel ledit milieu est un milieu liquide.

4. Le dispositif de la revendication 3, dans lequel ledit milieu est un milieu aqueux.

5. Le dispositif de la revendication 1, dans lequel ladite irradiation non ionisante préalable du milieu est réalisée par utilisation d'une antenne de Lecher (7) placée au-dessus d'un volume dudit milieu et tenue à la main (11) par l'une de ses branches (8).

6. Le dispositif de la revendication 1, dans lequel ladite irradiation non ionisante préalable du milieu est réalisée par utilisation d'une source (13) de rayonnement cohérent (12) associée à des moyens (14, 15) pour coopérer avec la main (11) d'un opérateur.

7. Le dispositif de la revendication 1, dans lequel ledit récipient a la forme d'au moins un tube allongé fermé à ses deux extrémités.

8. Le dispositif de la revendication 7, dans lequel ledit récipient a la forme de deux tubes parallèles disposés côte à côte.

9. Le dispositif de la revendication 7, dans lequel la longueur dudit tube ou desdits tubes est comprise entre 10 et 25 cm.

## Claims

1. A device for providing protection against the biological effects of non-ionizing electromagnetic radiation, in particular the radiation emitted by apparatuses having a display screen, the device being characterized in that it comprises a receptacle (1) designed to be placed in the proximity of the source of said radiation, the receptacle containing a medium (6) that has been subjected to prior non-ionizing irradiation and that contains a metal salt or a combination of metal salts at a maximum concentration of 20 mg of salt(s) per 100 liters of medium, said receptacle being made of a material that is non-ferromagnetic and that is chemically inert relative to said medium and to said salt.

2. The device of claim 1 in which said salt is a salt of an alkali metal or of an alkaline-earth metal.

3. The device of claim 1, in which said medium is a liquid medium.

4. The device of claim 3, in which said medium is an aqueous medium.

5. The device of claim 1, in which said prior non-ionizing irradiation of the medium is performed by using a Lecher antenna (7) placed over a volume of said medium and held in the hand (11) by one of its branches (8).

6. The device of claim 1, in which said prior non-ionizing irradiation of the medium is performed by using a source (13) of coherent radiation (12) associated with means (14, 15) for co-operating with the hand (11) of an operator.

7. The device of claim 1, in which said receptacle is in the form of at least one elongate tube that is closed at both ends.

8. The device of claim 7, in which said receptacle is in the form of two parallel tubes placed side by side.

9. The device of claim 7, in which the length of said tube or said tubes lies in the range 10 cm to 25 cm.

## Patentansprüche

1. Vorrichtung zum Schutz gegen die biologischen Auswirkungen von elektromagnetischen nicht-ionisierenden Strahlungen, insbesondere denjenigen, die durch Geräte mit einem Anzeigebildschirm emittiert werden,
dadurch gekennzeichnet, daß
sie einen Behälter (1) aufweist, der dazu bestimmt ist, in der Nähe der Quelle der Strahlungen angeordnet zu werden, und der ein Mittel (6) enthält, das zuvor einer nicht-ionisierenden Bestrahlung unterworfen worden ist, und ein metallisches Salz oder eine Kombination von metallischen Salzen, die in diesem Mittel mit einer maximalen Konzentration von 20 mg Salz(en) pro 100 Liter Mittel enthalten sind, wobei der Behälter aus einem nicht-ferromagnetischen und bezüglich des Mittels und des Salzes chemisch neutralen Material ist.

2. Vorrichtung gemaß Anspruch 1, bei der das Salz ein Salz aus einem alkalischen oder erdalkalischen Metal ist.

3. Vorrichtung gemaß Anspruch 1, bei der das Mittel ein flüssiges Mittel ist.

4. Vorrichtung gemaß Anspruch 3, bei der das Mittel ein wäßriges Mittel ist.

5. Vorrichtung gemaß Anspruch 1, bei der die vorangehende nicht-ionisierende Bestrahlung des Mittels durch Verwendung einer Lecher-Antenne (7) ausgeführt wird, die über einem Volumen mit dem Mittel angeordnet wird und mit der Hand (11) an einem ihrer Zweige (8) gehalten wird.

6. Vorrichtung gemaß Anspruch 1, bei der die vorangehende nicht-ionisierende Bestrahlung des Mittels durch Verwendung einer Quelle (13) von kohärenter Bestrahlung (12) ausgeführt wird, die Einrichtungen (14, 15) zugeordnet ist, um mit der Hand (11) eines Benutzes zusammenzuwirken.

7. Vorrichtung gemäß Anspruch 1, bei der das Gefäß die Form zumindest einer langgestreckten Röhre aufweist, die an ihren beiden Enden verschlossen ist.

8. Vorrichtung gemäß Anspruch 7, bei der das Gefäß die Form von zwei parallelen Röhren aufweist, die Seite an Seite angeordnet sind.

9. Vorrichtung gemäß Anspruch 7, bei der die Länge der Röhre oder der Röhren zwischen 10 und 25 cm beträgt.
